# EUROPÄISCHE PATENTANMELDUNG

(11) **EP 2 636 751 A2**
(43) Veröffentlichungstag der Anmeldung: **11.09.2013**
(21) Anmeldenummer: 13171066.7
(22) Anmeldetag: 19.03.2010
(51) Int. Cl.: C12Q 1/54, G01N 33/52, G01N 33/66, G01N 21/63

(54) **Verfahren zum Bestimmen einer Körperflüssigkeit**

(30) Priorität: 20.03.2009 EP 09004009
(62) Teilanmeldung aus: 10711144.5
(71) Anmelder: Roche Diagnostics GmbH, 68305 Mannheim (DE); F. Hoffmann-La Roche AG, 4070 Basel (CH)
(72) Erfinder: Haar, Hans-Peter, 69168 Wiesloch (DE); Horn, Carina, 68647 Biblis (DE)
(74) Vertreter: Bittner, Thomas L.

(57) **Zusammenfassung**

Es ist ein Verfahren zum Bestimmen einer Körperflüssigkeit offenbart, bei dem eine Probe einer Körperflüssigkeit auf einen Nachweisbereich eines für eine Bestimmung der Körperflüssigkeit konfigurierten Testelementes aufgegeben wird, wobei der Nachweisbereich mit einem für die Körperflüssigkeit sensitiven Reagens beladen ist, während einer Nachweisreaktion in dem Nachweisbereich Messsignale erfasst werden, indem das Testelement gemessen wird, ein oder mehrere Zustandsparameter für den Nachweisbereich erfasst werden, indem mittels einer Zustandsmessung Messsignale für ein oder mehrere im Nachweisbereich und / oder benachbart hierzu angeordnete Funktionselemente gemessen werden, und für die während einer Nachweisreaktion erfassten Messsignale unter Berücksichtigung der für den einen oder die mehreren Zustandsparameter erfassten Messsignale eine Messwertkorrektur ausgefiihrt wird.

## Beschreibung

Die Anmeldung betrifft ein Verfahren zum Bestimmen einer Körperflüssigkeit, insbesondere für eine Blutzuckerbestimmung.

### Hintergrund

Testelemente werden genutzt, um Körperflüssigkeiten zu bestimmen, insbesondere zum Nachweis einer Körperflüssigkeit als solcher oder eines von ihr enthaltenen Bestandteils. Beispielsweise erfolgt das Bestimmen der Körperflüssigkeit in Verbindung mit einem solchen Testelement mit einer optischen Auswertung. Verschiedene optische Messparameter können hierbei ausgewertet werden, beispielsweise die Lichtemission in Form einer Fluoreszenz oder einer Phosphoreszenz oder die Lichtabsorption. Die optischen Messmethoden haben sich für einige Testelemente in Form von Teststreifen, insbesondere in Verbindung mit der Untersuchung kleiner Blutvolumina, als zweckmäßig erwiesen. Auch bei ergonomisch ausgeführten Teststreifen können sie Vorteil bringend eingesetzt werden.

Zum Nachweis der Körperflüssigkeit oder eines von ihr enthaltenen Bestandteils verfügen die Testelemente regelmäßig über einen so genannten Nachweisbereich, in welchem üblicherweise eine chemische Nachweisreaktion der Körperflüssigkeit oder eines ihrer Bestandteile mit einem im Nachweisbereich des Testelementes vorhandenen Reaktionssystem stattfindet, so dass ein oder mehrere Reaktionsprodukte anschließend messtechnisch detektiert werden können, insbesondere mit Hilfe einer optischen Messung.

Um anhand der Auswertung des Testelementes die korrekte Schlussfolgerung hinsichtlich der Bestimmung der Körperflüssigkeit zu ziehen, sind Qualität und Genauigkeit des Messprozesses von besonderer Bedeutung. So ist der Einsatz bestimmter Testelemente nur in hierfür vorgegebenen Temperaturbereichen erlaubt, da nur in diesen Temperaturbereichen eine funktionsgerechte Bestimmung der Körperflüssigkeit mit ausreichender Sicherheit gewährleistet ist. Bei einer Blutzuckerbestimmung ist beispielsweise der Hämatokritwert und somit dessen Einfluss auf die Messung temperaturabhängig. In Verbindung mit Testelementen zur Blutzuckerbestimmung wurden deshalb Messungen und hieraus abgeleitete Korrekturen bei der Auswertung der Messung auf Basis der Elektrochemie oder der Amperometrie mittels der Impedanzmethode vorgeschlagen.

Das Dokument EP 1 411 346 A2 beschreibt ein Verfahren zur Erkennung einer Unterdosierung eines analytischen Testelements sowie ein System und ein Testelement, die zur Erkennung einer Unterdosierung geeignet sind.

Das Dokument DE 10 2004 051 830 A1 beschreibt ein System für einen Lumineszenznachweis eines Analytes in einer flüssigen Probe (z. B. Blut).

In dem Dokument EP 0 819 943 A2 wird ein Analysesystem zur Auswertung von analytischen Testelementen beschrieben.

### Zusammenfassung

Aufgabe ist es, ein verbessertes Verfahren zum Bestimmen einer Körperflüssigkeit, insbesondere für eine Blutzuckerbestimmung, zu schaffen, mit dem die Genauigkeit der Körperflüssigkeitsbestimmung optimiert ist. Hierbei soll zusätzlicher Aufwand bei der Auswertung eines Testelementes möglichst minimiert oder ganz vermieden werden.

Diese Aufgabe wird durch ein Verfahren zum Bestimmen einer Körperflüssigkeit nach dem unabhängigen Anspruch 1 gelöst. Vorteilhafte Ausgestaltungen sind Gegenstand von abhängigen Unteransprüchen.

Es ist die Möglichkeit geschaffen, auf direkte Art und Weise Zustandsinformationen für den Nachweisbereich des Testelementes effizient experimentell zu gewinnen. Die Zustandsinformationen können dann auf verschiedene Art und Weise ausgewertet werden. Beispielsweise kann anhand der Zustandsinformation entschieden werden, ob ein Testelement aufgrund seiner Eigenschaften im Nachweisbereich überhaupt noch für eine Bestimmung der Körperflüssigkeit geeignet ist oder nicht. Werden Abweichungen von einem als Normal- oder Mindestmaß definierten Zustand festgestellt, kann das Testelement verworfen werden. Es ist eine Berücksichtigung der Zustandsinformation bei der eigentlichen Bestimmung der Körperflüssigkeit vorgesehen in Form einer Korrektur erfasster Messsignale. Auf diese Weise können beispielsweise unterschiedliche Reaktionsverläufe für die Nachweisreaktion berücksichtigt werden. Die zum Erfassen des wenigstens einen Zustandsparameters für den Nachweisbereich genutzte Zustandsmessung, mit welcher das Funktionselement auswertbar ist, findet in Verbindung mit der Bestimmung der Körperflüssigkeit selbst statt zum Zweck der erwähnten Messkorrektur.

Des Weiteren ist ein Testelement zum Bestimmen einer Körperflüssigkeit, insbesondere für eine Blutzuckerbestimmung, offenbart, mit einem Nachweisbereich, welcher mit einem für die Körperflüssigkeit sensitiven Reagens beladen ist, wobei im Nachweisbereich und / oder benachbart hierzu ein zum Erfassen wenigstens eines Zustandsparameters für den Nachweisbereich mittels einer Zustandsmessung auswertbares Funktionselement angeordnet ist.

Bei dem Funktionselement kann es sich um einen Stoff, eine Verbindung oder ein Stoffgemisch handeln. Das Funktionselement kann auf dem Testelement ausschließlich im Nachweisbereich, ausschließlich benachbart hierzu oder sowohl im Nachweisbereich als auch benachbart hierzu angeordnet sein.

Für die Zustandsmessung stehen verschiedenste als solche bekannte Messmethoden zur Verfügung, auf die je nach Ausführung des von dem Testelement enthaltenen Funktionselementes zurückgegriffen werden kann.

Eine Weiterbildung sieht vor, dass das Funktionselement ein gegenüber einer Nachweisreaktion beim Bestimmen der Körperflüssigkeit reaktionschemisch inertes Funktionselement ist. Bei dieser Ausführungsform wird das Funktionselement durch die Nachweisreaktion bei der Bestimmung der Körperflüssigkeit selbst nicht chemisch verändert. Alternativ oder ergänzend hierzu kann vorgesehen sein, dass auch das Funktionselement seinerseits die Reaktionschemie der Nachweisreaktion nicht beeinflussend ausgeführt ist.

Bei einer zweckmäßigen Ausgestaltung kann vorgesehen sein, dass das Funktionselement ein für einen Reaktionsparameter der Nachweisreaktion beim Bestimmen der Körperflüssigkeit sensitives Funktionselement ist. Ein Reaktionsparameter der Nachweisreaktion, der anhand einer experimentellen Auswertung des Funktionselementes überprüft werden kann, ist beispielsweise die Temperatur vor, während oder nach der Nachweisreaktion im Nachweisbereich. Wird als Funktionselement zum Beispiel ein lichtemittierendes Funktionselement verwendet, so kann dessen Lichtemissionsverhalten während der Nachweisreaktion als Indikator für die Temperatur im Nachweisbereich ausgewertet werden. Beispielsweise kann der zeitliche Verlauf des Abklingens einer Fluoreszenz oder einer Phosphoreszenz nach einer vorherigen Anregung als Temperaturindikator ausgewertet werden. Als nutzbare Funktionselemente sind in diesem Zusammenhang beispielhaft zu nennen, so genannte Europiumkomplexe wie Europiumchelate. Bei solchen Europiumkomplexen hängt das Fluoreszenzabklingverhalten von der Temperatur ab (vgl. zum Beispiel Katagiri et al., J. Alloys and Comp., 408-412, 809-812 (2006); Wolfbeis et al., Anal. Chem., 78, 5094-5101 (2006); Khalil et al., Sens. Act. B. Chemical, 96, 304-314 (2003); Wolfbeis et al., Analyst, 132, 507-511 (2007)). Besonders vorteilhaft kann in diesem Zusammenhang vorgesehen sein, die Europiumkomplexe in eine Polymermatrix einzubetten, beispielsweise eine Polyvinylmethylketonmatrix. Auf diese Weise sind Polymerkügelchen herstellbar, die beim Herstellen des Testelementes in den Nachweisbereich und / oder benachbart hierzu eingebracht werden können. Je nach verwendetem Funktionselement können so ein oder mehrere Reaktionsparameter für die Nachweisreaktion beim Bestimmen der Körperflüssigkeit experimentell mittels einer Messung in direkter Art und Weise ausgewertet werden.

Eine alternative Ausführungsform sieht vor, dass das Funktionselement ein für einen Testelementparameter sensitives Funktionselement ist. Bei dieser Ausführungsform ist das Testelement alternativ oder ergänzend zu anderen Ausgestaltungen mit Hilfe des Funktionselementes konfiguriert, derart, dass Parameter des Testelementes mittels der Zustandsmessung für das Funktionselement erfasst werden können. Hierbei ist beispielsweise die funktionelle Integrität des Testelementes überprüfbar. Zum Beispiel kann die Schichtdicke einer oder mehrerer Schichten mit Hilfe des in die Schichten eingelagerten Funktionselementes anhand der Zustandsmessung ausgewertet werden. So kann in einer Ausführungsform das Funktionselement ein lichtemittierendes Element sein. Im Rahmen der Zustandsmessung kann anhand der Intensität des gemessenen Lichtemission die Dicke einer Schicht rückgeschlossen werden, in welcher sich das Funktionselement auf dem Testelement befindet. Auch kann vorgesehen sein, dass eine gemessene Lichtintensität im Rahmen der Zustandsmessung mit Vergleichswerten verglichen wird, die für das Testelement zu einem früheren Zeitpunkt, beispielsweise unmittelbar nach der Herstellung, erfasst wurden. Auf diese Weise ist feststellbar, ob in der Schicht, in welcher sich das Funktionselement befindet, eine die Schichtdicke beeinflussende Veränderung stattgefunden hat, die das Testelement beispielsweise unbrauchbar machen kann. Wenn eine Veränderung festgestellt wird, die das Testelement noch in einem brauchbaren Zustand belässt, kann die Schichtdickeninformation zur Korrektur von Messergebnissen genutzt werden, die im Rahmen der Bestimmung der Körperflüssigkeit erfasst werden.

Es kann vorgesehen sein, dass das Funktionselement ein zum Erfassen des wenigstens einen Zustandsparameters für den Nachweisbereich mittels einer optischen Zustandsmessung auswertbares Funktionselement ist. Auf diese Weise ist die Zustandsmessung mit Hilfe einer Messtechnik auswertbar, nämlich einer optischen Messung, die in Auswertegeräten für solche Testelemente bereits zur Verfügung steht, beispielsweise zur Detektion eines Farbumschlages beim Bestimmen der Körperflüssigkeit. Das Funktionselement kann konfiguriert sein, Licht in Form einer Fluoreszenz oder einer Phosphoreszenz abzugeben. Auch kann vorgesehen sein, die im Rahmen der optischen Zustandsmessung erfassten optischen Messsignale zeitaufgelöst, spektral aufgelöst und / oder intensitätsabhängig zu erfassen. Je nach verwendetem Funktionselement und hiermit auswertbarer Zustandsinformation kann die optische Zustandsmessung angepasst werden.

Bei einer Ausgestaltung kann vorgesehen sein, dass das Funktionselement ein zum Erfassen des wenigstens einen Zustandsparameters für den Nachweisbereich mittels einer zeitaufgelösten optischen Zustandsmessung auswertbares Funktionselement ist. Eine Weiterbildung kann vorsehen, dass das Funktionselement ein zum Erfassen des wenigstens einen Zustandsparameters für den Nachweisbereich mittels einer intensitätsabhängigen optischen Zustandsmessung auswertbares Funktionselement ist. Die zeitaufgelöste optische Zustandsmessung und die intensitätsabhängige optische Zustandsmessung können bedarfsweise mit einer Spektralauflösung kombiniert werden, bei der die erfassten optischen Messsignale wellenlängenabhängig ausgewertet werden.

Eine andere Weiterbildung sieht vor, dass das Funktionselement wenigstens teilweise mit dem Reagens vermischt ist. Mit Hilfe des Vermischens von Funktionselement und Reagens wird das Funktionselement zumindest teilweise in das Reagens eingebettet, was eine direkte Erfassung von Zustandsparametern für den Nachweisbereich weiter unterstützt.

Bei einer zweckmäßigen Ausgestaltung kann vorgesehen sein, dass das Funktionselement im Nachweisbereich oder benachbart hierzu in mehreren räumlich voneinander getrennten Bereichen angeordnet ist. Auf diese Weise ist es ermöglicht, Zustandsparameter für unterschiedliche räumliche Bereiche im Nachweisbereich oder benachbart hierzu zu erfassen. In diesem Zusammenhang kann für die zur Auswertung des Funktionselementes genutzte Messtechnik vorgesehen sein, ein oder mehrere Dektektoren zu verwenden, um die mehreren räumlich voneinander getrennten Bereiche experimentell zu untersuchen. Im Fall einer optischen Zustandsmessung können mehrere optische Sensoren vorgesehen sein, zum Beispiel in Form von Sensordioden.

Eine weitere Ausführungsform sieht vor, dass im Nachweisbereich oder benachbart hierzu ein zum Erfassen wenigstens eines weiteren Zustandsparameters für den Nachweisbereich mittels einer weiteren Zustandsmessung auswertbares weiteres Funktionselement angeordnet ist. Die Nutzung unterschiedlicher Funktionselemente kann dazu beitragen, unterschiedliche Zustandsparameter für den Nachweisbereich zu erfassen. Alternativ oder ergänzend kann aber auch vorgesehen sein, dass mit Hilfe unterschiedlicher Funktionselemente gleiche Zustandsparameter für den Nachweisbereich erfasst werden, so dass diese Zustandsinformation auf verschiedene Art und Weise und somit mit höherer Zuverlässigkeit gewonnen wird. Das weitere Funktionselement kann bedarfsweise den vorangehend beschriebenen verschiedenen Ausgestaltungsmöglichkeiten für das Funktionselement entsprechend gebildet sein. Im Fall einer optischen Zustandsmessung und hierfür geeigneter Funktionselementen kann zum Beispiel vorgesehen sein, dass die unterschiedlichen Funktionselemente Licht verschiedener Farben emittieren, so dass dieses auf einfache Art und Weise mit Hilfe einer Wellenlängenfilterung getrennt erfasst und ausgewertet werden kann.

Eine Fortbildung sieht einen zumindest im Nachweisbereich gebildeten Mehrschichtaufbau vor. In dem Mehrschichtaufbau kann das Funktionselement in einer oder in mehreren Schichten angeordnet sein. Auch kann in einer Ausführungsform vorgesehen sein, dass in eine oder in mehrere Schichten unterschiedliche Funktionselemente eingebettet sind. Auf diese Weise ist das Testelement je nach Aufgabenstellung konfigurierbar, um Zustandsinformationen für den Nachweisbereich zu erfassen.

Nachfolgend werden Ausgestaltungen des Verfahrens zum Bestimmen einer Körperflüssigkeit näher erläutert.

Bei einer Ausgestaltung kann vorgesehen sein, dass als Zustandsparameter ein Reaktionsparameter für eine Nachweisreaktion beim Bestimmen der Körperflüssigkeit in dem Nachweisbereich erfasst wird. Beispielsweise kann mit Hilfe einer optischen Zustandsmessung für ein auf diese Art und Weise auswertbares Testelement die Temperatur für die Nachweisreaktion im Nachweisbereich direkt erfasst werden.

Eine Weiterbildung kann vorsehen, dass als Zustandsparameter ein Testelementparameter für das Testelement erfasst wird. Testelementparameter, die auf diese Art und Weise erfasst werden können, sind beispielsweise die Dicke von Schichten des Testelementes, die mechanische Integrität von Bereichen des Testelementes oder auch Schichtdickenvariationen. Es kann vorgesehen sein, ein für ein Funktionselement erfasstes Messsignal auf verschiedene Art und Weise auszuwerten, um unterschiedliche Zustandsparameter zu erfassen. Im Fall einer optischen Zustandsmessung, bei der optische Messsignale für in dem Testelement angeordnete Funktionselement erfasst werden, kann das optische Signal, zum Beispiel in Form eines Fluoreszenz- oder eines Phosphoreszenzsignals, zeitaufgelöst und intensitätsabhängig erfasst werden, um so zum Beispiel Informationen über die Temperatur im Nachweisbereich einerseits und die Schichtdicke auf dem Testelement andererseits zu gewinnen.

Eine andere Weiterbildung sieht vor, dass die Messsignale mit Vergleichssignalen verglichen werden. Als Vergleichssignale stehen beispielsweise Messwerte zur Verfügung, die für das Testelement zu einem früheren Zeitpunkt und / oder unter anderen Umgebungsbedingungen erfasst wurden. Beispielsweise kann für das Testelement, wenn es sich um ein trockenchemisches Testelement handelt, eine Messung im trockenen Ausgangszustand erfolgen, zum Beispiel unmittelbar nach der Herstellung. Diese Messsignale können dann verglichen werden mit aktuell erfassten Messsignalen, wenn auf das Testelement eine Probe der zu bestimmenden Körperflüssigkeit aufgegeben ist oder wenn dieses beabsichtigt ist. In Verbindung mit Reaktionsparametern für die Nachweisreaktion können die Messsignale mit vorgegebenen Werten verglichen werden, die erfüllt sein müssen, um die korrekte Bestimmung der Körperflüssigkeit sicherzustellen. Liegt ein Reaktionsparameter außerhalb der vorgegebenen Werte, beispielsweise in Form einer zu hohen oder zu niedrigen Temperatur, kann in dem Auswertegerät für das Testelement hierauf reagiert werden, zum Beispiel mittels einer Kühlung oder einer Heizung für das Testelement. So werden für typische Testelemente beispielsweise Einsatzbereiche zwischen etwa 15°C und etwa 35°C vorgegeben. Mittels der hier vorgeschlagenen Techniken sind Testelemente geschaffen, die für ein Einsatzgebiet im Bereiche zwischen etwa 5°C und etwa 50°C konfiguriert sind.

In Verbindung mit Testelementparametern kann bei einer Abweichung von Vergleichswerten, zum Beispiel aufgrund einer Chargenschwankung, ein anderer Verlauf der Nachweisreaktion erwartet werden, was bei der Auswertung der Bestimmung der Körperflüssigkeit dann berücksichtigt werden kann.

Bei einer zweckmäßigen Ausgestaltung kann vorgesehen sein, dass als Reaktion auf das Erfassen des einen oder der mehreren Zustandsparameter für den Nachweisbereich die Nachweisreaktion beim Bestimmen der Körperflüssigkeit beeinflussende Reaktionsparameter verändert werden. Das Erfassen des einen oder der mehreren Zustandsparameter kann vor dem Bestimmen der Körperflüssigkeit erfolgen, um dann für die Nachweisreaktion in dem Auswertegerät für das Testelement entsprechende Parameter einzustellen. Aber auch ein Erfassen der Zustandsparameter während der Nachweisreaktion kann ergänzend oder alternativ hierzu vorgesehen sein.

Eine weitere Ausführungsform sieht vor, dass die Zustandsmessung als eine optische Messung ausgeführt wird, bei der optische Messsignale gemessen werden. Die optische Messung wird üblicherweise ausgeführt, indem Anregungslicht einer bestimmten Farbe oder einer Mischfarbe auf den Nachweisbereich des Testelementes eingestrahlt wird, wodurch das eine oder die mehreren Funktionselemente angeregt werden und selbst Licht emittieren, zum Beispiel in Form von Fluoreszenz oder Phosphoreszenz. Das emittierte Licht kann dann mit Hilfe eines oder mehrerer Detektoren spektral aufgelöst, zeitaufgelöst und / oder intensitätsabhängig erfasst werden, um unterschiedliche Zustands- oder Messinformationen zu gewinnen. Eine Lichtemission kann jedoch auch angeregt werden, indem der Nachweisbereich mit einem elektrischen Strom beaufschlagt wird. Auch eine Chemolumineszenz kann zur Gewinnung von Zustandsinformationen ausgewertet werden.

### Beschreibung einiger Ausführungsbeispiele

Im Folgenden werden Ausführungsbeispiele unter Bezugnahme auf Figuren einer Zeichnung näher erläutert. Hierbei zeigen:
- Fig. 1: eine schematische Darstellung eines Mehrschichtaufbaus eines Testelementes zum Bestimmen einer Körperflüssigkeit,
- Fig. 2: eine schematische Darstellung einer Messanordnung zum optischen Messen eines Testelementes zum Bestimmen einer Körperflüssigkeit,
- Fig. 3: eine grafische Darstellung eines Fluoreszenzverlaufes in Abhängigkeit von der Zeit für verschiedene Glucosekonzentrationen,
- Fig. 4: eine grafische Darstellung von Fluoreszenzkurven in Abhängigkeit von der Zeit für unterschiedliche Temperaturen,
- Fig. 5: eine grafische Darstellung des gemessenen Fluoreszenzabklingverhaltens in Abhängigkeit von der Zeit bei verschiedenen Temperaturen für den Europiumkomplex in Fig. 7,
- Fig. 6: eine grafische Darstellung für aus den Messergebnissen in Fig. 5 ermittelte Abklingzeiten in Abhängigkeit von der Temperatur,
- Fig. 7: die Strukturformel eines Europiumkomplex,
- Fig. 8: eine grafische Darstellung von experimentell bestimmten Glucosekonzentrationen in Blutproben in Abhängigkeit von bekannten Referenzkonzentrationen und
- Fig. 9a, 9b: Strukturformeln von weiteren als Funktionselement nutzbaren Europiumkomplexen.

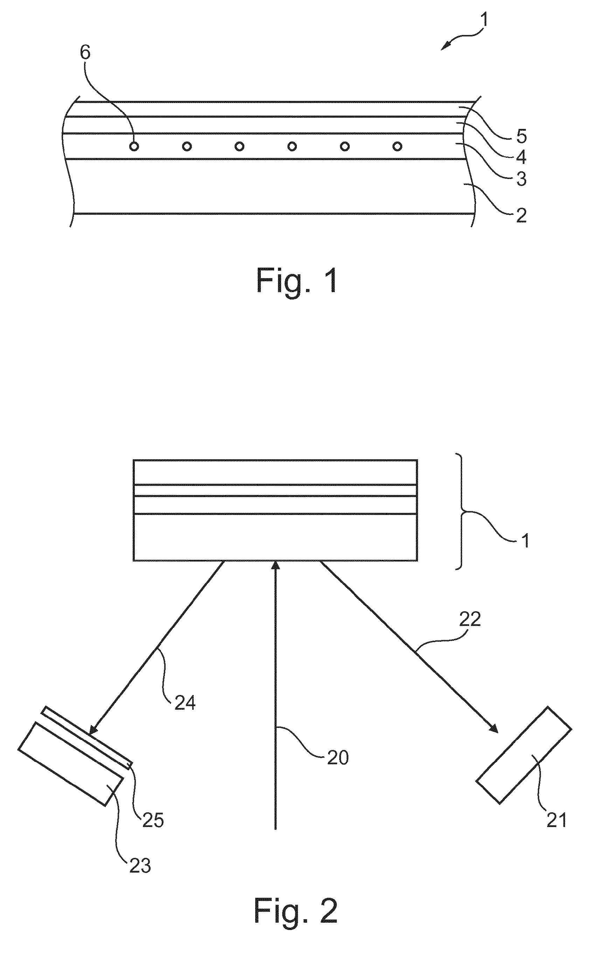

Fig. 1 zeigt eine schematische Darstellung eines Mehrschichtaufbaus eines Testelementes 1, welches für eine Bestimmung einer Körperflüssigkeit konfiguriert ist, insbesondere eine Blutzuckerbestimmung. Auf einem lichtdurchlässigen Trägersubstrat 2, welches in einer Ausführungsform von einer Trägerfolie aus Polycarbonat mit einer Dicke von etwa 30 µm bis etwa 150 µm gebildet ist, befindet sich eine aktive Schicht 3, welche ihrerseits die Reaktionschemie für die Bestimmung der Körperflüssigkeit enthält, beispielsweise ein Reagens in Form eines enzymatischen Systems. Die aktive Schicht 3 ist beispielsweise mit einer Dicke von etwa 10 µm gebildet. In der aktiven Schicht 3 findet die Nachweisreaktion statt, zum Beispiel in Form einer Farbreaktion, welche dann in einem für das Testelement hergerichteten Auswertegerät mittels einer optischen Messung auswertbar ist. Verschiedene Reaktionssysteme sind für das Bestimmen einer Körperflüssigkeit als solche und hinsichtlich ihrer Auswertbarkeit, die bevorzugt optisch erfolgt, bekannt, weshalb hierauf nicht weiter eingegangen wird.

Oberhalb der aktiven Schicht 3 ist eine weitere Schicht 4 gebildet, die in einer Ausführungsform Pigmente enthält, zum Beispiel in Form von TiO2 oder ZrO2. Die weitere Schicht 4 kann beispielsweise eine Dicke von etwa 10 µm aufweisen. Bei einer möglichen Konfiguration des Testelementes 1 für eine Blutbestimmung, dient die weitere Schicht 4 zum Abtrennen der Erythrozyten. Das eingelagerte Pigment reflektiert bei der optischen Auswertung des Testelementes eingestrahltes Messlicht diffus. Schließlich ist oberhalb der weiteren Schicht 4 eine Netzschicht 5 hergestellt, die zum Spreiten des Blutes dient.

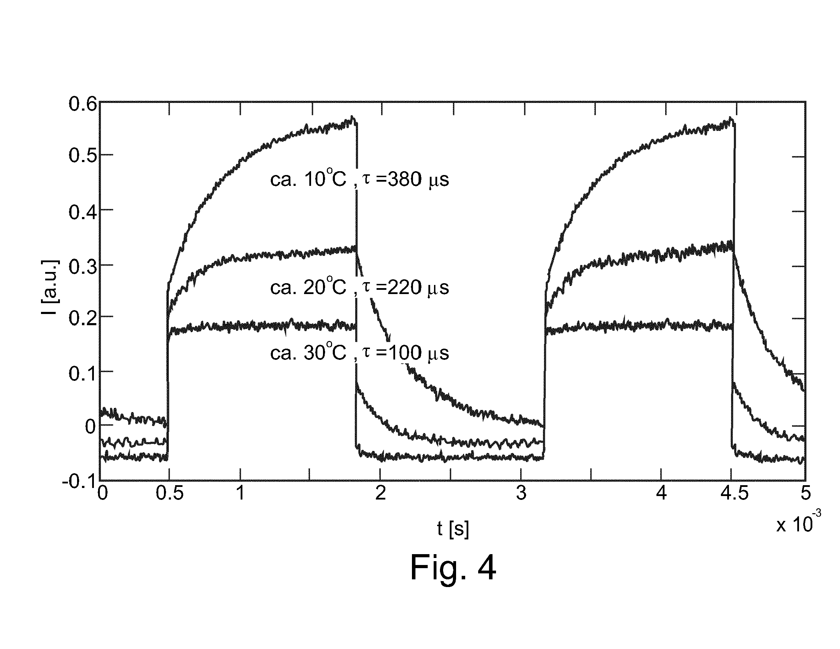

In die aktive Schicht 3 sind Funktionselemente 6 eingelagert, bei denen es sich in einer möglichen Ausführungsform um Kügelchen aus einem Polymer handelt, in welche Europiumkomplexe eingebettet sind. Zwei Ausführungsformen für derartige Europiumkomplexe sind in Fig. 4a und 4b gezeigt. Die Europiumkomplexe dienen als Funktionselemente, die bei optischer Anregung Licht emittieren, welches mit einem oder mehreren optischen Sensoren erfasst werden kann.

Fig. 2 zeigt eine schematische Darstellung einer Messanordnung zum optischen Messen eines Testelementes zum Bestimmen einer Körperflüssigkeit. Auf ein Testelement 1, welches in der dargestellten Ausführungsform der Beschreibung in Verbindung mit Fig. 1 entsprechend ausgebildet ist, wird Messlicht 20 eingestrahlt, welches eine Wellenlänge von 365 nm aufweist. Über einen Messkanal 21 wird diffus an dem Testelement 1 reflektiertes Licht 22 detektiert. Die Signalerfassung erfolgt zeitaufgelöst. Detektiert wird der zeitliche Intensitätsverlauf des diffus reflektierten Lichtes 22 für verschiedene Glucosekonzentrationen auf dem Testelement 1.

Über einen weiteren Empfangskanal 23 wird Fluoreszenzlicht 24 der Europiumkomplexe zeitaufgelöst und intensitätsabhängig detektiert. Das Messlicht 20 wird mit Hilfe eines Kantenfilters 25 Wellenlängen selektiv ausgeblendet. Beispielsweise kann ein Kantenfilter verwendet werden, der für Wellenlängen > 450 nm durchlässig ist. Die mit Hilfe des weiteren Empfangskanals 23 gemessenen Fluoreszenzsignale werden dann mit Hilfe einer elektronischen Auswerteeinrichtung (nicht dargestellt) ausgewertet. Beispielsweise kann das Fluoreszenzabklingverhalten ausgewertet werden. Hierbei werden eine oder mehrere Abklingzeiten für die mit Hilfe des Messlichtes 1 angeregte Fluoreszenz der Europiumkomplexe in dem Testelement 1 ermittelt. Alternativ oder ergänzend kann die Phasenverschiebung zwischen dem Messlicht 20 und dem Fluoreszenzlicht 24 mittels Nutzung der Lock-in-Technik bestimmt werden. Aus der Phasenverschiebung lassen sich dann mit Hilfe üblicher Techniken Informationen über das Fluoreszenzabklingverhalten ableiten.

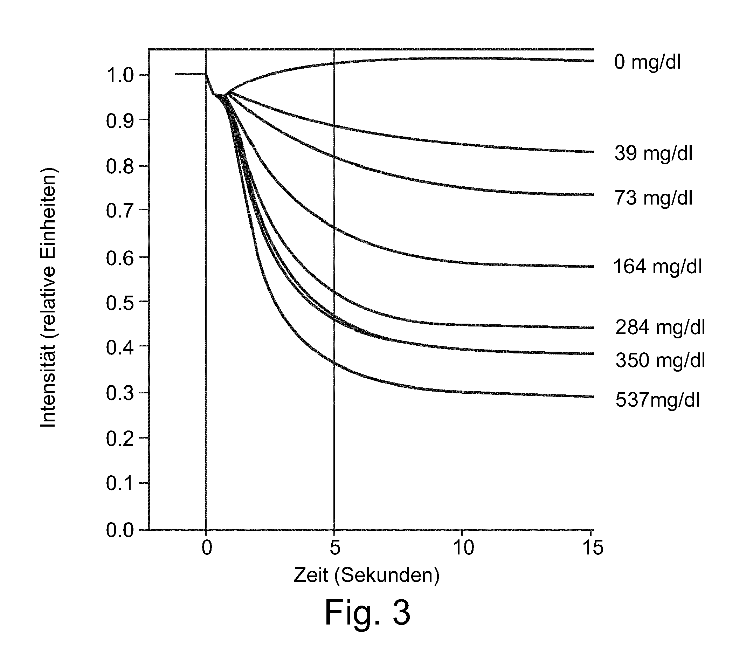

Die Fig. 3 eine grafische Darstellung eines Intensitätsverlaufes in Abhängigkeit von der Zeit für verschiedene Glucosekonzentrationen. Dargestellt ist der Intensitätsverlauf für die diffuse Reflexion beim optischen Untersuchen eines mit einer Blutprobe beladenen Testelementes unter Verwendung der Messanordnung in Fig. 2. Hierbei wurde das Testelement zum Zeitpunkt Null mit einer Blutprobe beladen, so dass das zunächst trockene Testelement nass wird. Hierauf startet die Reaktion der Bildung einer Spezies, für die dann die optische Untersuchung durchgeführt wird, beispielsweise mittels Erfassen der diffusen Reflexion, die auch als Remission bezeichnet wird. Dargestellt sind mehrere Intensitätsverläufe für unterschiedliche Glucosekonzentrationen.

Fig. 4 zeigt eine grafische Darstellung von Fluoreszenzkurven in Abhängigkeit von der Zeit für unterschiedliche Temperaturen. Es ergibt sich, dass bei unterschiedlichen Temperaturen, nämlich etwa 10°C, etwa 20°C und etwa 30°C, das Fluoreszenzverhalten in seinem zeitlichen Verlauf verschieden ist. Die Messsignale wurden nach einer optischen Anregung mit Anregungslicht von etwa 360 nm erfasst. Der Detektor befand sich senkrecht oberhalb der Probe in einem Abstand von etwa 3 mm. Das unterschiedliche Fluoreszenzverhalten ermöglicht somit einen Rückschluss auf die Temperatur in der Umgebung der lichtemittierenden Elemente. Für das in Fig. 1 dargestellte Testelement 1 bedeutet dies, dass mit Hilfe der Funktionselemente 6 der vom Nachweisbereich umfassten aktiven Schicht 3 direkt die Temperatur ermittelt werden kann, die für die Nachweisreaktion beim Bestimmen der Körperflüssigkeit in diesem Bereich vorhanden ist. In Abhängigkeit von der Auswertung des Zeitverhaltens der Fluoreszenz und der hieraus geschlossenen Temperaturverhältnisse könne dann bedarfsweise die Bedingungen für die Bestimmung der Körperflüssigkeit in dem Auswertegerät eingestellt oder verändert werden.

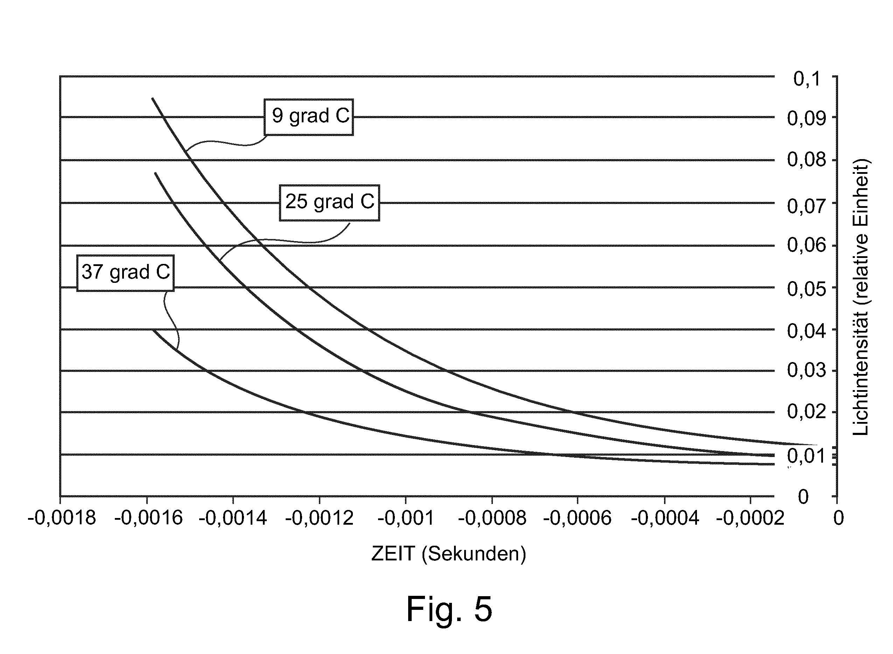

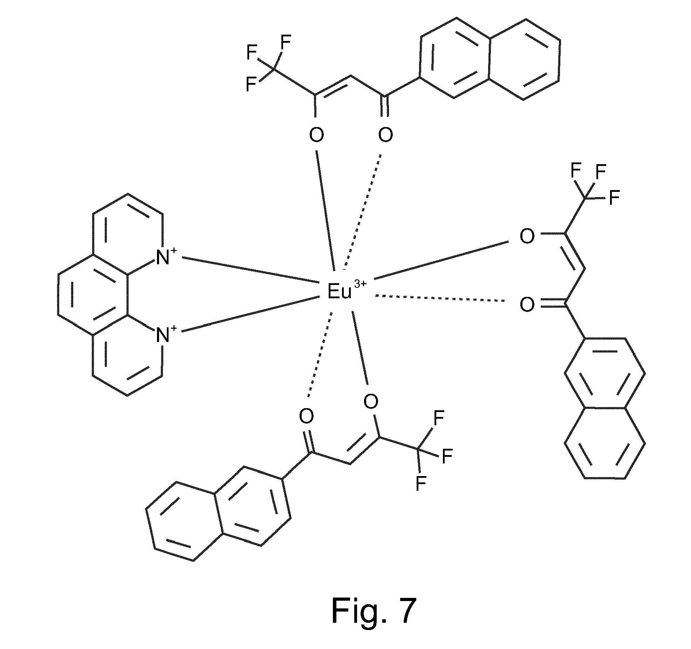

Fig. 5 zeigt eine grafische Darstellung des gemessenen Fluoreszenzabklingverhaltens in Abhängigkeit von der Zeit bei verschiedenen Messtemperaturen für den als Funktionselement nutzbaren Europiumkomplex in Fig. 7. Gemessen wurde die Intensität des Fluoreszenzlichtes des Europiumkomplexes bei 9°C (obere Kurve), 25°C (mittlere Kurve) und 37°C (untere Kurve).

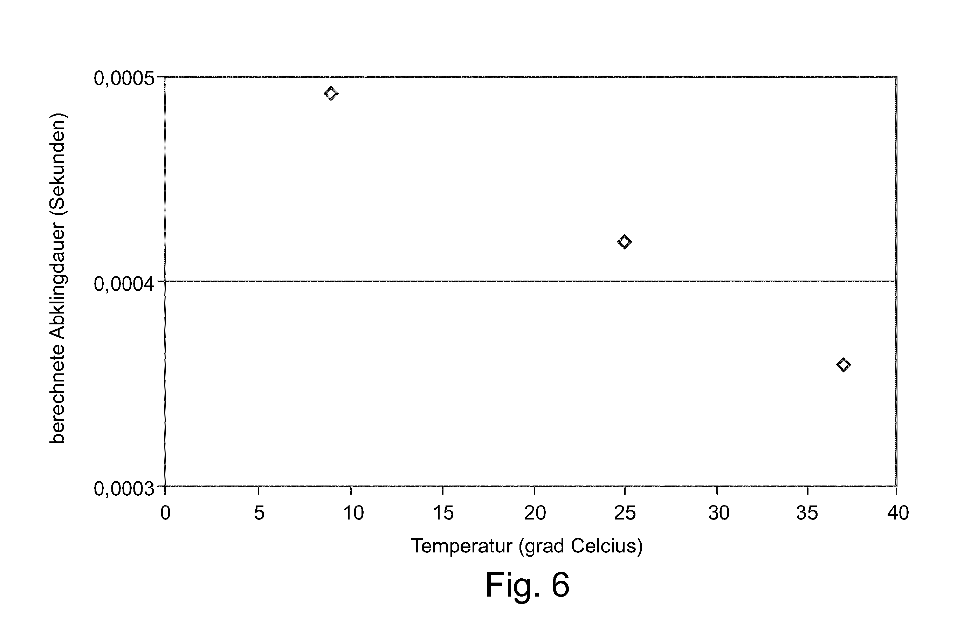

Fig. 6 zeigt eine grafische Darstellung für aus den Messergebnissen in Fig. 5 ermittelte Abklingzeiten in Abhängigkeit von der Temperatur. Mit zunehmender Temperatur wird Fluoreszenzabklingzeit kürzer.

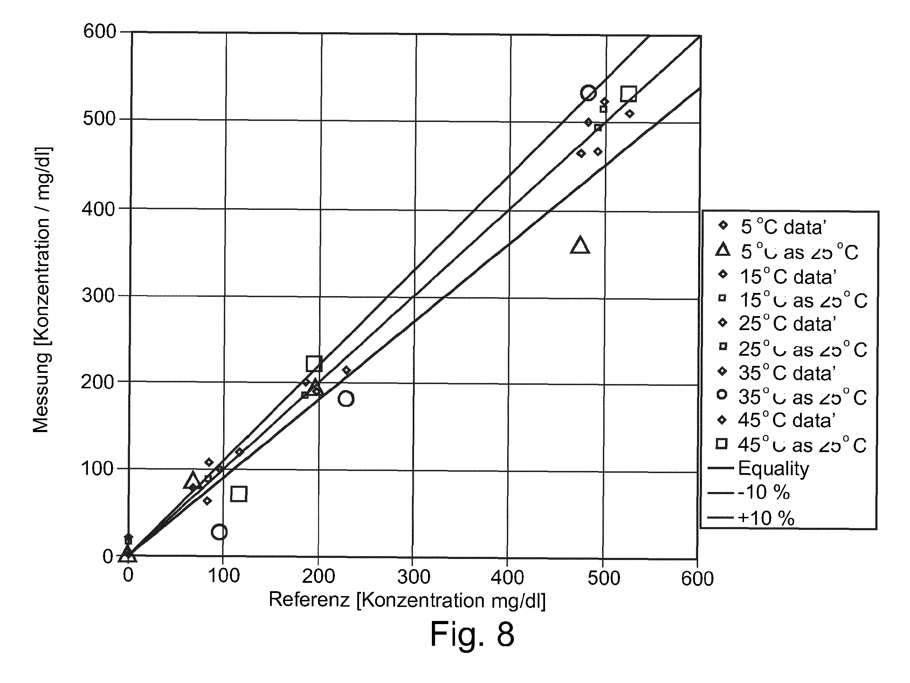

Fig. 8 zeigt eine grafische Darstellung von experimentell bestimmten Glucosekonzentrationen in Blutproben in Abhängigkeit von bekannten Referenzkonzentrationen.

Die großen Symbole (Dreiecke, Vierecke, Kreise) in Fig. 8 zeigen unkorrigierte Glucosekonzentrationswerte für die untersuchten Blutproben. Demgegenüber wurde für die mit kleinen Symbolen dargestellten Glucosekonzentrationswerte eine Messkorrektur dahingehend vorgenommen, dass die tatsächlich vorhandene Umgebungstemperatur bei der Glucosebestimmung berücksichtigt wird. Die tatsächliche Umgebungstemperatur wurde der vorangehend unter Bezugnahme auf die Fig. 5 und 6 erläuterten Methode entsprechend ermittelt aus dem Fluoreszenzverhalten der beigegebenen Europiumkomplexe. Für die mit einem großen Dreieck in Fig. 8 dargestellten Glucosekonzentrationswerte bedeutet dies, dass die ursprünglich angenommene Umgebungs- oder Messtemperatur von 25°C auf 5°C korrigiert wurde. Für die mit einem großen Kreissymbol gekennzeichneten Glucosekonzentrationswerte erfolgte die Korrektur von 25°C auf 35°C, und für die mit dem großen Vierecksymbol gezeigten Glucosekonzentrationswerte erfolgte die Korrektur von 25°C auf 45°C. Es ergibt sich, dass die korrigierten Glukosekonzentrationswerte deutlich näher an den mittleren Kurvenverlauf in Fig. 8 liegen.

Für die rechnerische Korrektur können unterschiedliche Verfahren eingesetzt werden; zum Beispiel können experimentell ermittelte Stützstellen ermittelt werden, zwischen denen beispielsweise linear interpoliert wird, oder es können einfach nichtlineare Näherungsfunktionen ermittelt werden, die mit wenigen Stützstellen auskommen.

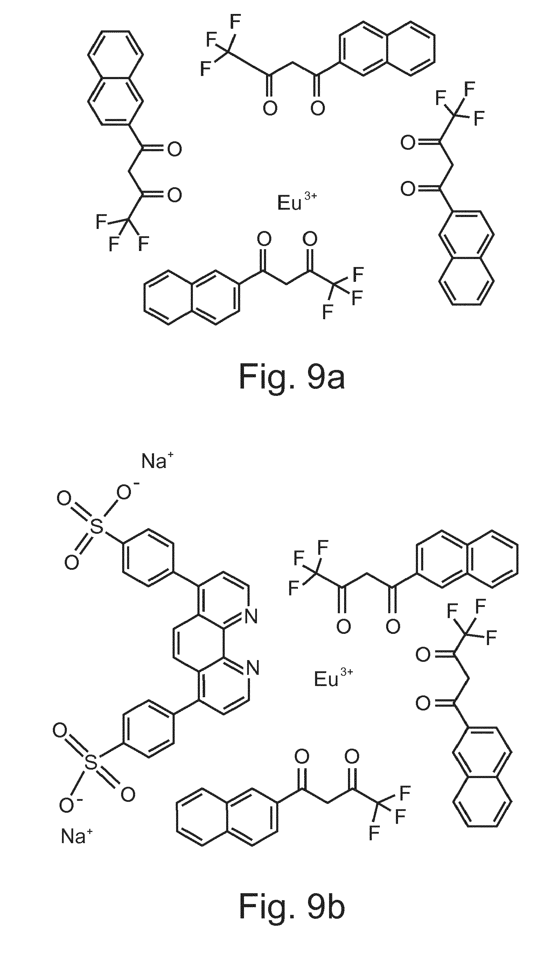

Fig. 9a und 9b zeigen Strukturformeln von weiteren als Funktionselement nutzbaren Europiumkomplexen.

## Patentansprüche

1. Verfahren zum Bestimmen einer Körperflüssigkeit bei dem
- eine Probe einer Körperflüssigkeit auf einen Nachweisbereich eines für eine Bestimmung der Körperflüssigkeit konfigurierten Testelementes aufgegeben wird, wobei der Nachweisbereich mit einem für die Körperflüssigkeit sensitiven Reagens beladen ist,
- während einer Nachweisreaktion in dem Nachweisbereich Messsignale erfasst werden, indem das Testelement gemessen wird,
- ein oder mehrere Zustandsparameter für den Nachweisbereich erfasst werden, indem mittels einer Zustandsmessung Messsignale für ein oder mehrere im Nachweisbereich und / oder benachbart hierzu angeordnete Funktionselemente gemessen werden, und
- für die während einer Nachweisreaktion erfassten Messsignale unter Berücksichtigung der für den einen oder die mehreren Zustandsparameter erfassten Messsignale eine Messwertkorrektur ausgeführt wird.

2. Verfahren nach Anspruch 1, **dadurch gekennzeichnet, dass** als Zustandsparameter ein Reaktionsparameter für die Nachweisreaktion beim Bestimmen der Körperflüssigkeit in dem Nachweisbereich erfasst wird.

3. Verfahren nach Anspruch 1 oder 2, **dadurch gekennzeichnet, dass** als Zustandsparameter ein Testelementparameter für das Testelement erfasst wird.

4. Verfahren nach einem der vorangehenden Ansprüche, **dadurch gekennzeichnet, dass** die Messsignale mit Vergleichssignalen verglichen werden.

5. Verfahren nach einem der vorangehenden Ansprüche, **dadurch gekennzeichnet, dass** als Reaktion auf das Erfassen des einen oder der mehreren Zustandsparameter für den Nachweisbereich die Nachweisreaktion beim Bestimmen der Körperflüssigkeit beeinflussende Reaktionsparameter verändert werden.

6. Verfahren nach einem der vorangehenden Ansprüche, **dadurch gekennzeichnet, dass** die Zustandsmessung als eine optische Messung ausgeführt wird, bei der optische Messsignale gemessen werden.

7. Verfahren nach Anspruch 6, **dadurch gekennzeichnet, dass** die Zustandsmessung mittels einer zeitaufgelösten optischen Zustandsmessung ausgeführt wird.

8. Verfahren nach Anspruch 6 oder 7, **dadurch gekennzeichnet, dass** die Zustandsmessung mit einer intensitätsabhängigen optischen Zustandsmessung ausgeführt wird.

9. Verfahren nach einem der vorangehenden Ansprüche, **dadurch gekennzeichnet, dass** das eine oder die mehreren Funktionselemente gegenüber der Nachweisreaktion beim Bestimmen der Körperflüssigkeit reaktionschemisch inert sind.

10. Verfahren nach einem der Ansprüche 1 bis 8, **dadurch gekennzeichnet, dass** das eine oder die mehreren Funktionselemente gegenüber der Nachweisreaktion beim Bestimmen der Körperflüssigkeit reaktionschemisch sensitiv sind.

11. Verfahren nach einem der vorangehenden Ansprüche, **dadurch gekennzeichnet, dass** das eine oder die mehreren Funktionselemente für einen Testelementparameter sensitiv sind.
